(19) Europäisches Patentamt
European Patent Office
Office européen des brevets

(11) **EP 1 784 144 B1**

(12) **EUROPEAN PATENT SPECIFICATION**

(45) Date of publication and mention
of the grant of the patent:
**28.12.2011 Bulletin 2011/52**

(51) Int Cl.:
***A61F 2/06*** *(2006.01)*     ***A61F 2/88*** *(2006.01)*

(21) Application number: **05779569.2**

(22) Date of filing: **04.08.2005**

(86) International application number:
**PCT/US2005/027592**

(87) International publication number:
**WO 2006/017586 (16.02.2006 Gazette 2006/07)**

(54) **VASCULAR PROSTHESIS HAVING IMPROVED FLEXIBILITY AND NESTED CELL DELIVERY CONFIGURATION**

GEFÄSSPROTHESEN MIT VERBESSERTER FLEXIBILITÄT UND EINER ABGABEKONFIGURATION MIT VERSCHACHTELTEN ZELLEN

PROTHESE VASCULAIRE PRESENTANT UNE MEILLEURE FLEXIBILITE ET CONFIGURATION D'ADMINISTRATION ALVEOLAIRE IMBRIQUEE

(84) Designated Contracting States:
**AT BE BG CH CY CZ DE DK EE ES FI FR GB GR HU IE IS IT LI LT LU LV MC NL PL PT RO SE SI SK TR**

(30) Priority: **04.08.2004 US 911435**

(43) Date of publication of application:
**16.05.2007 Bulletin 2007/20**

(73) Proprietor: **NovoStent Corporation
Mountain View, CA 94043 (US)**

(72) Inventors:
• **HOGENDIJK, Michael
Santa Clara, CA 95054 (US)**
• **LEOPOLD, Eric
Redwood City, CA 94062 (US)**
• **MILES, Alexander
Fremont, CA 94555 (US)**
• **HUYNH, Tim
Santa Clara, CA 95051 (US)**

(74) Representative: **Vossius & Partner
Siebertstrasse 4
81675 München (DE)**

(56) References cited:
WO-A-98/41169     WO-A-2004/058100
WO-A1-83/00997     US-A- 4 760 849
US-A- 4 760 849     US-A- 5 716 396
US-B1- 6 174 305

**Description**

Technical Field

The present invention relates to an implantable vascular prosthesis configured for use in a wide range of applications, and more specifically, a vascular prosthesis having improved flexibility and at least partially nested cells in a reduced delivery configuration.

Background Art

Vascular stenting has become a practical method of reestablishing blood flow to a patient's diseased vasculature. Today there are a wide range of intravascular prostheses on the market for use in the treatment of aneurysms, stenoses, and other vascular irregularities. Balloon expandable and self-expanding stents are well known for restoring patency in a stenosed vessel, e.g., after an angioplasty procedure, and the use of coils and stents are known techniques for treating aneurysms.

Previously-known self-expanding stents generally are retained in a contracted delivery configuration using an outer sheath, and then self-expand when the sheath is retracted. Such stents commonly have several drawbacks, for example, the stents may experience large length changes during expansion (referred to as "foreshortening") and may shift within the vessel prior to engaging the vessel wall, resulting in improper placement. Additionally, many self-expanding stents have relatively large delivery profiles because the configuration of their struts limits further compression of the stent. Accordingly, such stents may not be suitable for use in smaller vessels, such as cerebral vessels and coronary arteries.

For example, PCT Publication WO 00/62711 to Rivelli describes a stent comprising a helical mesh coil having a plurality of turns and including a lattice having a multiplicity of pores. The lattice is tapered along its length. In operation, the plurality of turns are wound into a reduced diameter helical shape, then constrained within a delivery sheath. The delivery sheath is retracted to expose the distal portion of the stent and anchor the distal end of the stent. As the delivery sheath is further retracted, the subsequent individual turns of the stent unwind to conform to the diameter of the vessel wall.

The stent described in the foregoing publication has several drawbacks. For example, due to friction between the turns and the sheath, the individual turns of the stent may bunch up, or overlap with one another, when the delivery sheath is retracted. In addition, once the sheath is fully retracted, the turns may shift within the vessel prior to engaging the vessel wall, resulting in improper placement of the stent. Moreover, the stent pattern may not be sufficiently flexible to allow the stent to be rolled to a small delivery profile diameter.

In an attempt to control foreshortening, some previously-known stent designs axially nest adjacent turns of the stent in the contracted delivery state, as described in U.S. Patents 5,575,816 and 5,906,639, both to Rudnick et al. Axial nesting as described in those patents is only possible if the helical portion of the stent comprises a relatively narrow element, such as the sinusoidal wire form depicted in those patent. Accordingly, axial nesting is not a practical solution where the helical portion of the stent has a substantial width relative to the longitudinal axis of the stent.

Still other stent designs attempt to address foreshortening issues by overlapping adjacent turns of the helical portion in the contracted delivery state, such as described in U.S. Patent 6,425,915 to Khosravi et al. One drawback of such an arrangement, however, is stacking of adjacent helical turns increases the delivery profile of the stent.

WO 2004/058100 A discloses a vascular prosthesis and methods of use. The prosthesis comprises one or more helical sections coupled to one or more anchoring sections having a generally zig-zag or cell-like configuration. The prosthesis is configured to conform to a vessel wall without substantially remodelling the vessel. The prosthesis is further configured to be precisely deployed in a vessel without shifting or foreshortening during deployment.

In view of these drawbacks of previously known devices, it would be desirable to provide an example of an apparatus and a method (not part of the invention) for an implantable vascular prosthesis comprising a stent that exhibits a high degree of flexibility in a reduced delivery profile.

It also would be desirable to provide another example of an apparatus and a method (not part of the invention) for a vascular prosthesis having a body portion featuring cells that do not interfere with each other when the body portion is rolled to a reduced delivery configuration.

It further would be desirable to provide a further example of an apparatus and a method (not part of the invention) for a vascular prosthesis having cell configurations that provide substantially linear helical features throughout the pattern, wherein the linear features may include angular changes and/or hinge points to provide a vascular prosthesis having increased flexibility in the reduced delivery configuration and good radial strength.

It also would be desirable to provide a method for a vascular prosthesis having a helical mesh configuration that permits adjacent turns of the vascular prosthesis to be wound down in an overlapping manner without substantially increasing the delivery profile of the prosthesis.

It still further would be desirable to provide a method for a vascular prosthesis having a helical mesh configuration that permits at least partial axial nesting of adjacent turns of the vascular prosthesis without substantially increasing the delivery profile of the prosthesis.

Summary of the disclosure

In view of the foregoing, an exemplified apparatus and a method, not part of the invention, for an implantable vascular prosthesis comprising a stent that exhibits a high degree of flexibility in a reduced delivery profile is disclosed.

An another exemplified apparatus and a method, not part of the invention, for a vascular prosthesis having a body portion featuring cells that do not interfere with each other when the body portion is rolled to a reduced delivery configuration is disclosed.

A further apparatus and a method, not part of the invention, for a vascular prosthesis having cell configurations that provide substantially linear helical features throughout the pattern, wherein the linear features may include angular changes and/or hinge points is disclosed.

An object of the present invention is to provide a method according to the appended claims for a vascular prosthesis that has a substantially small delivery configuration, thereby allowing the prosthesis to be used in smaller vessels.

Another object of this invention is to provide a method according to the appended claims for a vascular prosthesis having a helical mesh configuration that permits adjacent turns of the vascular prosthesis to be wound down in an overlapping manner without substantially increasing the delivery profile of the prosthesis.

A further object of this invention is to provide a method according to the appended claims for a vascular prosthesis having a helical mesh configuration that permits at least partial axial nesting of adjacent turns of the vascular prosthesis without substantially increasing the delivery profile of the prosthesis.

These and other objects of the present invention are accomplished by providing an implantable vascular prosthesis having improved flexibility, comprising a helical body portion capable of assuming a reduced delivery configuration and an expanded deployed configuration. The helical body portion comprises a cell configuration that provides increased flexibility and at least partial nesting of overlapping adjacent turns in the reduced delivery configuration. The cell configuration preferably comprises one or more substantially parallel struts that extend helically for the length of the helical body portion. More preferably, the cell configuration comprises a linear series of cells that are interconnected by hinged articulations.

The vascular prosthesis of an example not part of the invention may include cells having corners that define hinge elements, thereby allowing shape changes to occur in a planar fashion. Improved flexibility of the prosthesis in the reduced delivery configuration also may be achieved by providing a higher level of angularity among within the cell configuration. The radial force of the vascular prosthesis may be controlled by varying the placement of hinges within the cell configuration or by varying the angularity within the cell configuration. The radial force of the body portion also may be controlled by varying the angle that the cells are aligned along the longitudinal axis of the stent.

According to some examples, the individual cells that make up the body portion are sized so that the cell length is an integral fraction of the circumference of the body portion in the reduced delivery configuration. In other embodiments, the cells are dimensioned to provide a whole number of cells per reduced diameter circumference. The cells of adjacent turns of the body portion at least partially nest when overlapped in the reduced diameter circumference, thereby providing a reduced delivery volume. In addition, the cells may be configured to provide axial nesting by having the struts of one turn positioned along side of, rather than stacked directly atop, struts of an adjacent layer.

In a preferred example, the vascular prosthesis comprises a shape memory material, such as a nickel-titanium alloy, and includes a distal anchor section coupled to a proximal helical body portion having a plurality of turns. The cell configuration of the proximal portion includes features aligned substantially parallel to the helix of the helical body portion, such as angular changes and/or hinge points. By providing a cell configuration having a greater number of angular changes and hinge points, a vascular prosthesis may be obtained having greater flexibility and that provides nesting of adjacent turns in the reduced delivery configuration. Preferably, the cell configurations provide substantially linear helical components throughout the pattern.

Methods of using the vascular prosthesis also are provided.

Brief Description Of Drawings

Further features of the invention, its nature and various advantages will be more apparent from the accompanying drawings and the following detailed description of the preferred embodiments, in which:

FIG. 1 is a perspective view of a first example of a vascular prosthesis;
FIGS. 2A-2B are respectively, a perspective view of an alternative example of a vascular prosthesis and plan view of a portion of the unwound helical body portion;

FIG. 3 is a plan view of a portion of an unwound body portion of a further alternative example of a vascular prosthesis;

FIG. 4 is a perspective view of a distal portion of an inner member for a delivery catheter suitable for use with the vascular prosthesis;

FIG. 5 is a schematic view of overlapping adjacent turns of vascular prosthesis, according to the present invention;

FIG. 6 is an image of overlapping adjacent turns of a vascular prosthesis, according to the present invention;

FIG. 7 is a side view, partly in section, of a vascular prosthesis disposed within an illustrative delivery catheter;

FIGS. 8A-8G are side-sectional views depicting use of the apparatus of FIG. 7 to perform angioplasty and to delivery a vascular prosthesis;

FIG. 9 is a side view of a further alternative example of a vascular prosthesis;

FIG. 10 is a side view of yet another alternative example of a vascular prosthesis; and

FIG. 11 is a side view of a further alternative example of a vascular prosthesis.

Best Mode(s) for Carrying Out the Invention

The present invention is directed to a method of winding an implantable vascular prosthesis according to the appended claims, said prothesis configured for use in a wide range of applications, such as treating aneurysms, maintaining patency in a vessel, and allowing for the controlled delivery of therapeutic agents to a vessel wall. The prosthesis has a helical configuration that provides a substantially smaller delivery profile than previously-known devices. In a preferred example, the stent also includes a radially expandable distal portion joined to the helical body portion. Importantly, however, the principles of the present invention are advantageously applied to a stent comprising a helical body portion alone.

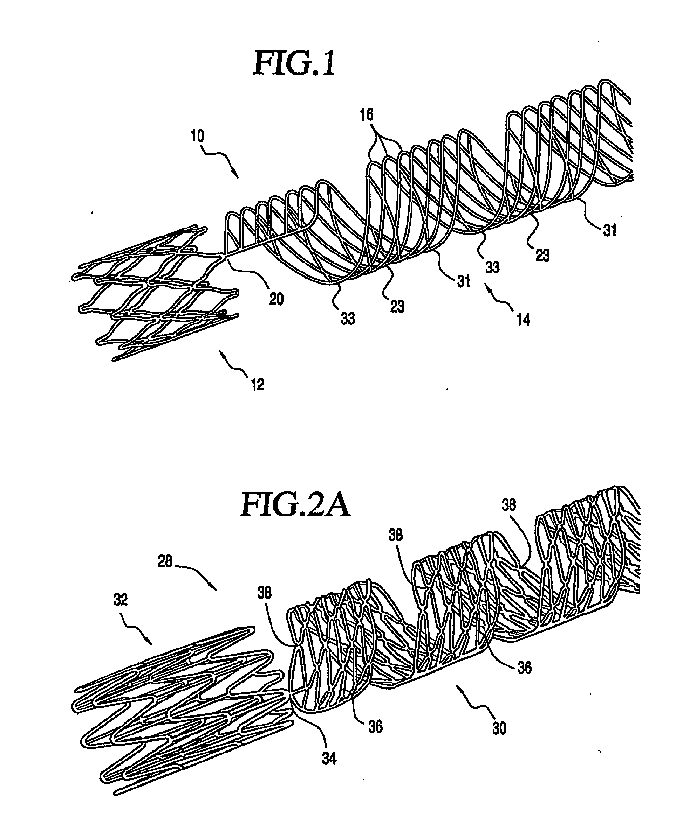

Referring to FIG. 1, vascular prosthesis 10 having improved flexibility and nesting capability, according to the principles of the present invention, is described. Vascular prosthesis 10 comprises helical body portion 14 and optional distal portion 12, each capable of assuming contracted and deployed states. In FIG. 1, helical body portion 14 and distal portion 12 are depicted in their deployed states. Helical body portion 14 is coupled to distal portion 12 at junction 20.

Body portion 14 comprises a plurality of turns 23 having a proximal edge 31 and distal edge 33. As used herein, proximal edge 31 is closer to the physician than distal edge 33 relative to the longitudinal axis of the delivery catheter when the prosthesis is delivered into a patient's vessel. Accordingly, as shown in FIG. 1, proximal edge 31 on each turn 23 of the prosthesis is disposed adjacent to distal edge 33 on an adjacent turn.

Vascular prosthesis 10 preferably is formed from a solid tubular member comprising a shape memory material, such as nickel-titanium alloy (commonly known in the art as Nitinol). The solid tubular member then is laser cut, using techniques that are per se known in the art, to a desired deployed configuration, as depicted in FIG. 1. An appropriate heat treatment, also known in the art, then may be applied to the vascular prosthesis while the device is held in a desired deployed configuration (e.g., on a mandrel). Treatment of the shape memory material allows vascular prosthesis 10 to self-deploy to the desired deployed configuration for purposes described hereinafter.

Still referring to FIG. 1, distal portion 12 is designed to be deployed from a stent delivery catheter first to fix the distal end of the stent at a desired known location within a vessel. Helical body portion 14 then may be deployed with great accuracy. In accordance with the principles of the present invention, helical body portion 14 comprises a cell configuration wherein a component of the cells, such as one or more struts 16, extends helically along the helical body portion without hinge points or angular changes. In general, struts that extend substantially parallel to the central axis of the stent provide greater stiffness in the reduced delivery configuration than do helical struts.

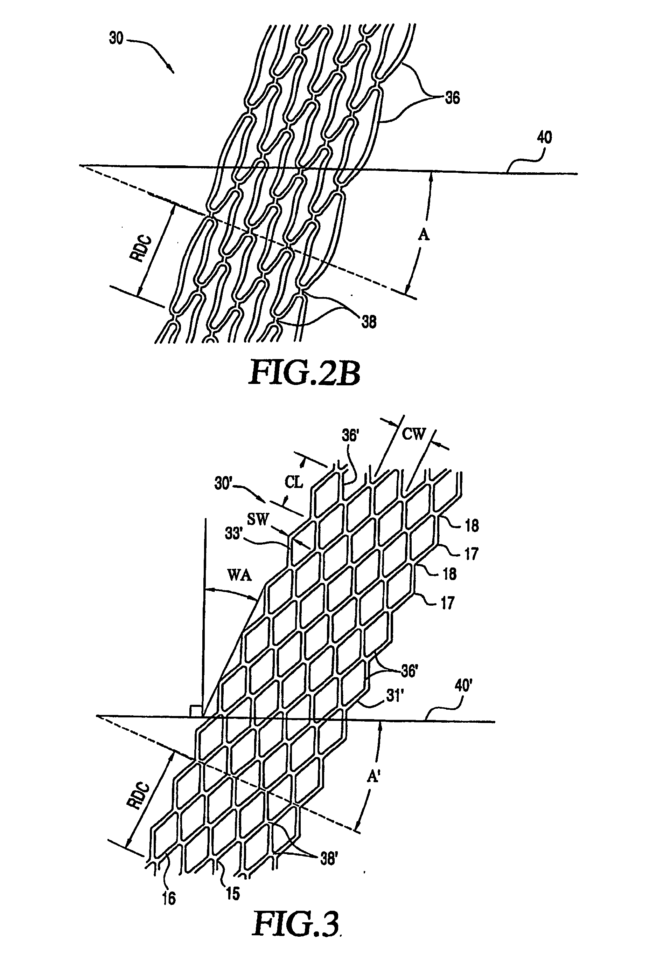

Referring now to FIGS. 2A and 2B, an alternative example of the vascular prosthesis is described. Vascular prosthesis 28 comprises helical body portion 30 and optional distal portion 32 coupled at junction 34. Helical body portion 30 comprises a series of cells 36 that are interconnected by hinges 38, wherein each hinge permits articulation of the stent. As depicted in FIG. 2B, cells 36 are substantially diamond-shaped, and aligned substantially end-to-end in a pattern that extends helically for the length of the stent. Adjacent cells are staggered, so that a line extending through the hinges of adjacent cells forms and angle A relative to the longitudinal axis 40 of the stent. It should be understood that cells 36 may have numerous other shapes (e.g., triangular, rhomboidal, pentagonal, curvilinear, etc.).

Vascular prosthesis 28 is provided with a high level of flexibility in the both the rolled for delivery state and deployed state by including more hinge points along the helically extending axis of the cells. Alternatively, a high degree of flexibility may be achieved by providing a higher level of angularity along the cell axis. As a further alternative, a combination of increased angularity and hinge points may be employed to achieve a desired degree of flexibility. By adding hinge points 38 and/or alternating the angularity of the struts (e.g., by adding curves or bends), the stiffness of the body portion may be lowered in the reduced delivery configuration.

It is therefore possible to control the flexibility of the vascular prosthesis through appropriate hinge placement and angularity. Further, the cells may be used to form patterns of variable metal concentrations and/or radial force values. The ability to vary the metal concentrations may be particularly advantageous in drug delivery applications, e.g., where the vascular prosthesis includes a coating of a bioactive substance, such as a drug that prevents restenosis.

The helical shape of the body portion inherently allows for a greater percentage of metal or scaffolding to be contained per unit length of stent. The vascular prosthesis comprises a helical body having a high degree of metal per unit length that retains high flexibility in the reduced delivery profile.

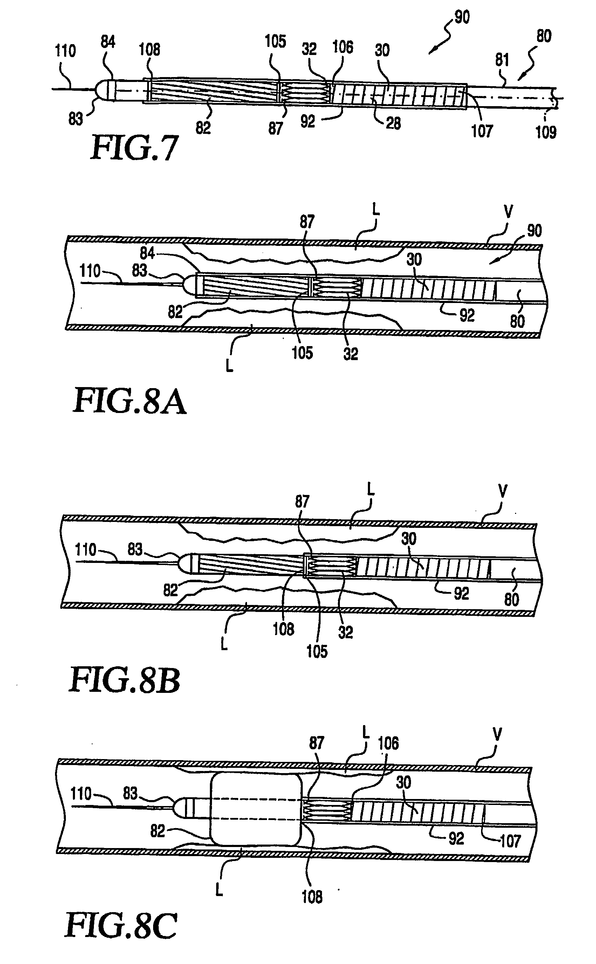

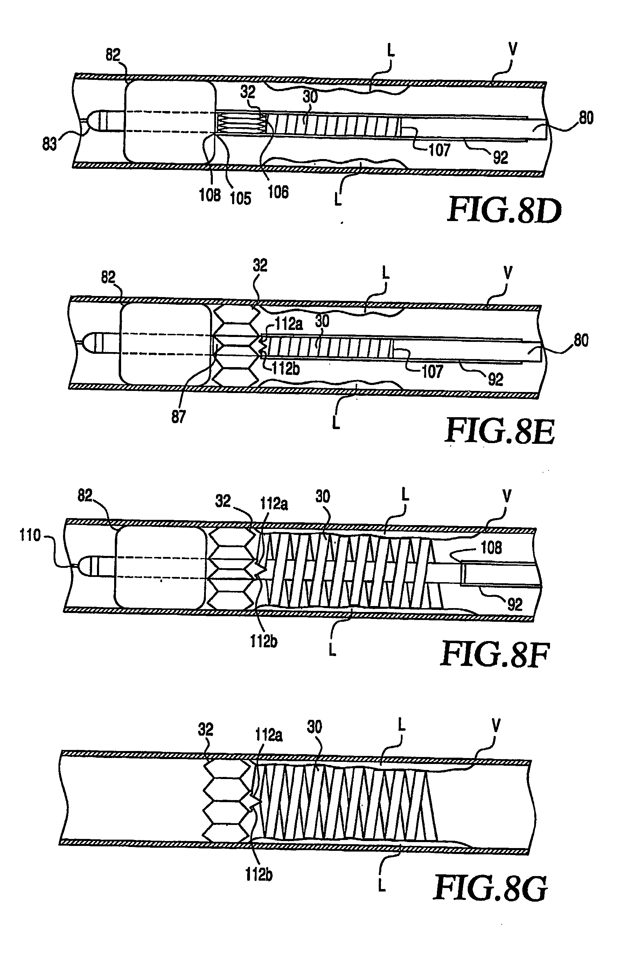

Generally, as the helical body is wound down to a reduced diameter delivery profile (see FIGS. 8), each successive wind overlaps the previous wind, thereby causing the stent to become stiffer. Stent flexibility in the reduced diameter delivery profile may be increased by imparting a pattern or cell configuration for the body portion that allows the rolled stent to articulate with limited friction or resistance.

FIG. 2B shows body portion 30 of the vascular prosthesis of FIG. 2A in an unwound, flattened configuration. Body portion 30 comprises a plurality of cells 36 interconnected by hinges 38 to permit increased flexibility. More particularly, in a preferred implementation, the corners of the cells are coupled by hinges 38 that allow for shape changes to occur in a planar fashion. To enhance flexibility of the stent in the reduced delivery profile, hinges 38 are aligned at an angle A relative to longitudinal axis 40 of the stent. As further depicted in FIG. 2B, the cells have a rounded or elongated diamond shape.

Referring now to FIG. 3, alternative body portion 30' comprises cells 36' that are diamond-shaped, but are not rounded or elongated like the cells of the example of FIGS. 2. As in the preceding embodiment, body portion 30' comprises staggered arrays of cells 36' that extend helically for the length of the helical body portion. In addition, hinges 38' that couple cells 36' together also are aligned at an angle A' relative to longitudinal axis 40' of the stent. Bends 15 and struts 16 of the cell pattern also define proximal edge 31' and distal edge 33' having a series of crests 17 and troughs 18. Cell 36' is characterized by cell length CL, cell width CW, strut width SW and wrap angle WA. Wrap angle WA represents the complementary angle to the angle formed between a line connecting crests 17 along distal edge 33' and longitudinal axis 40'.

Conventional vascular prostheses have a tendency to overlap and tangle when rolled to a reduced delivery configuration. In accordance with one aspect of the present invention, the cells of the vascular prostheses of the present invention are arranged so as to not interfere when rolled to the reduced delivery configuration. It is therefore important to design the cells so that the edge of a cell does not inadvertently engage the cells on adjacent winds when the vascular prosthesis is rolled for deployment. This is achieved by controlling the edge of the body portion as well as the width of the cells. Specifically, the more linear the edge of the body portion, the less likely the possibility of interference with other cells. In other words, longer, narrower cells (e.g., as depicted in FIG. 2B) are less likely to become interlocked with an adjacent layer of cells that would shorter, wider cells.

As discussed hereinabove with respect to FIG. 2B, hinges 38 of cells 36 preferably are aligned at an angle A with respect to longitudinal axis 40, preferably an oblique angle. The closer the hinges 38 are to being aligned perpendicular to longitudinal axis 40, the stiffer the helical body portion will be in the reduced delivery profile.

The vascular prostheses preferably include a "Reduced Delivery Circumference", or "RDC", corresponding to the circumference of the vascular prosthesis in the reduced delivery profile. The body portion features a cell pattern based on the RDC. More particularly, the cells that make up the cell pattern are dimensioned so that the length of a cell is an integral fraction of the RDC. Advantageously, a cell pattern that is dimensioned to provide a whole number of cells per RDC allows nesting of the cells along the axis of the vascular prosthesis in the reduced delivery profile. For example, referring to FIG. 2B, body portion 30 features a cell pattern comprising one cell per RDC. In FIG. 3, body portion 30' features a cell pattern comprising two cells per RDC. As would be appreciated by those of ordinary skill in the art, any whole number of cells per RDC may be employed in the cell pattern. Moreover, a cell pattern comprising a fractional number of cells per RDC also may be employed, as discussed below.

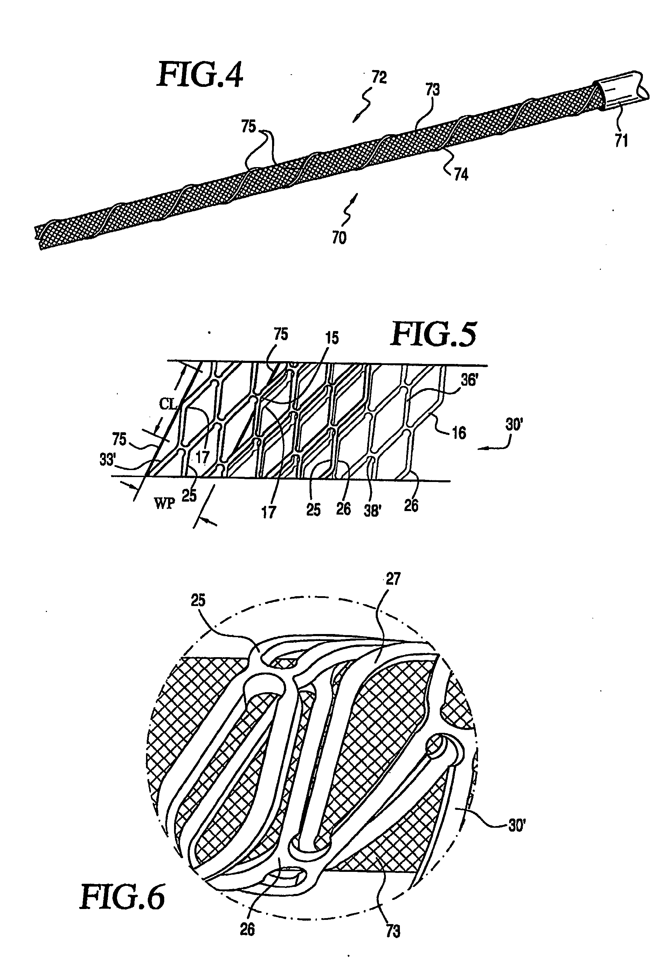

With respect to FIG. 4, delivery catheter 70, suitable for use with the vascular prosthesis is described. Delivery catheter 70 includes sheath 71 and inner member 72. Inner member 72 illustratively comprises braided wire tube 73 having helical wire 74 affixed to its outer surface to form a protruding helical guide 75, for example, using a biocompatible adhesive or solder. Guide 75 has a wrap pitch WP that is selected to guide wrapping of the prosthesis onto inner member 72 so as to control foreshortening of the vascular prosthesis during subsequent delivery.

Alternatively, helical wire 74 may be laminated to the outer surface of braided wire tube 73 using a polymeric layer, or inner member 72 itself may be formed by sandwiching a helical wire between inner and outer polymeric layers. Provision of guide 75 directly on the exterior surface of the inner member as in the embodiment of FIG. 4 permits a definable deployment length of the stent and also provides linear resistance to stent migration when sheath 71 is retracted during stent deployment.

During wrapping of a stent onto inner member 72, such as wrapping the embodiment of FIG. 1, either proximal edge 31 or distal edge 33 of the stent is abutted against guide 75, so that adjacent turns 23 of the stent overlap one another. Alternatively, braided tube 73 and helical wire 74 could be replaced with an inner member having an exterior surface including an integrally formed ridge. In addition, guide 75 could be formed by other features such as protrusions extending from the surface of inner member 72.

Referring now to FIG. 5, the nesting properties of a vascular prosthesis having the cell pattern of body portion 30' of FIG. 3 are described. FIG. 5 depicts the overlap of two adjacent turns of body portion 30' when wound in a proximal-to-distal direction on a portion of guide 75. Outer layer 25 partially overlaps underlying layer 26, with crests 17 of distal edge 33' urged against guide 75. Due to the proximal-to-distal wrapping of the prosthesis, outer layer 25 is disposed distal to underlying layer 26. Preferably, the cell configuration is selected such that bend 15 in outer layer 25 is in close proximity to crest of underlying layer 26 when wrapped onto inner member 72 having a predetermined wrap pitch WP. More preferably, the cell configuration is selected such that the areas of contact between layers occur at or near hinges 38'. As a result, at least partial nesting of the struts 16 of the overlapping cells occurs, with struts from outer layer 25 lying within the apertures of cells 36' of underlying layer 26. In this manner, the overall radial profile of the prosthesis is reduced relative to previously-known designs in which overlapping turns stack directly atop one another. Depending upon the wrap pitch and width of the turn, such nesting may be obtained with more than two layers.

The cells of the helical body of the vascular prosthesis are configured based on the RDC. More particularly, the cells are dimensioned so that cell length CL is either an integral fraction or multiply of the RDC. In the former case, the RDC divided by the length of the cells is an whole number; in the latter case, cell length CL is greater than the RDC. Such arrangements allow nesting of the cells of the prosthesis when wrapped along inner member 72 to the reduced delivery.profile. In addition, by reducing the number of cells per RDC, the number of struts that overlap may be reduced, further improving nesting of adjacent turns.

Preferably, the cells of the vascular prosthesis also are configured such that cell width CW is related to strut width SW, wrap pitch WP, and number of cells per wrap in the delivery.configuration according to the formula:

$$\mathtt{CW \;=\; (WP \pm SW) \;/\; (number\ of\ cells\ per\ wrap)}$$

For example, for wrap pitch WP of 2.032 mm (0.080"), strut width SW is 0.127 mm (0.005"), and a desired target of four cells per RDC, cell width CW should be selected as either 0.53975 mm (from (2.032 mm + 0.127 mm)/4) or 0.47625 mm (from (2.032 mm - 0.127 mm)/4) (0.02125" (from (0.080" + 0.005")/4) or 0.01875" (from (0.080" - 0.005")/4)). It should be appreciated that as the number of overlapping layers increases, increasing cell width CW facilitates in accommodating the additional, material. Preferably, cell width CW is related to strut width SW and number of overlapping layers by the expression:

CW > 2 * SW * (number of overlapping layers)

For example, if strut width SW is 0.127 mm (0.005") and there are two overlapping layers, cell width CW should be at least 0.508 mm (from 2 * 0.127 mm * 2 = 0.508 mm) (0.02" (from 2 * 0.005" * 2 = 0.02")). If cell length CW does not satisfy the above equation, a lesser degree of nesting may occur as overlapping struts are positioned atop an underlying layer.

Referring to FIG. 6, a vascular prosthesis having body portion 30' described above with respect to FIGS. 3 and 5 is shown wrapped onto an inner member of a delivery catheter. In FIG. 6, outer layer 25 is disposed atop underlying layer 26, which in turn is disposed atop underlying layer 27. Braided wire tube 73 of inner member 72 is visible beneath underlying layer 27. As shown in FIG. 6, the profile of outer layer 25 is comparable to that of underlying layer 27, providing a much smaller delivery profile than would be possible if the three adjacent turns were stacked directly atop one another.

Referring now to FIG. 7, an illustrative delivery catheter suitable for deploying the vascular prosthesis is described. In FIG. 7, inner member 80 of the delivery catheter is depicted carrying the inventive vascular prosthesis constrained on inner member 80 by retractable sheath 92. Inner member 81 includes polymer layer 87 that engages the distal end of the distal portion of vascular prosthesis 28 to prevent it from moving proximally when sheath 92 is retracted. Polymer layer 87 preferably is treated, e.g., by formulation, mechanical abrasion, chemically or by heat treatment, to make the polymer tacky or otherwise enhance the grip of the material. Polymer layer 87 may comprise a proximal shoulder of balloon 82, or alternatively may be formed and applied separately from balloon 82. As a yet further alternative, balloon 82 may be omitted, and polymer layer 87 may be disposed adjacent the distal end of the inner member.

Delivery catheter 90 is pre-loaded with vascular prosthesis 28 of the type shown in FIG. 2A, wherein the prosthesis is constrained between inner member 81 and sheath 92. Prosthesis 28 includes distal portion 32 that is engaged with polymer layer 87, and helical body portion 30 that is wrapped to a small diameter around the shaft of inner member 81. Sheath 92 restrains vascular prosthesis 28 against the shaft of inner member 81 until the sheath is retracted proximally. Balloon 82 is shown deflated and wrapped around the shaft of the inner member, in accordance with known techniques.

Sheath 92 is depicted in its insertion configuration, wherein the sheath extends over balloon 82 to a position just proximal of distal end 83. Delivery catheter 90 optionally may include radio-opaque marker bands 105, 106 and 107 disposed, respectively, on inner member 81 beneath the distal and proximal ends of distal portion 32 and at the proximal end of body portion 30. Sheath 92 also may include radio-opaque marker 108 disposed adjacent to its distal end. Delivery catheter 90 preferably includes guide wire lumen 109 that enables the delivery catheter to be slidably translated along guide wire 110.

In operation, delivery catheter 90 is advanced along a guide wire into a vessel containing a treatment area, e.g., plaque or a lesion. Positioning of the vascular prosthesis relative to the treatment area is confirmed using radio-opaque markers 84 and 105-107. Once the delivery catheter is placed in the desired location, sheath 92 is retracted proximally to permit vascular prosthesis 28 to deploy. Polymer layer 87 grips distal portion 32 of stent 28, and prevents distal portion 32 from being dragged proximally into engagement with helical body portion 30 during retraction of sheath 92. Instead, polymer section 87 grips distal portion 32 against axial movement, and permits the distal portion to expand radially outward into engagement with the vessel wall once the outer sheath is retracted.

In addition, as described with respect to FIGS. 8 hereinbelow, either before or after distal portion 32 is expanded into engagement with the vessel wall, balloon 82 is expanded to contact the vessel wall. Balloon 82 therefore anchors distal end 83 of delivery catheter 90 relative to the vessel wall, so that no inadvertent axial displacement of the delivery catheter arises during proximal retraction of the sheath to release distal portion 32 or helical body portion 30 of the vascular prosthesis 28.

Referring now to FIGS. 8, a method (not part of the invention) of using delivery catheter 90 of FIG. 7 to perform angioplasty and deliver vascular prosthesis 28 are described. Vascular prosthesis 28 is disposed in its delivery configuration with distal portion 32 compressed around inner member 80 and retained by sheath 92. Distal portion 32 of prosthesis 28 is disposed in contact with polymer layer 87 to prevent relative axial movement therebetween, as described above.

As shown in FIG. 8A, delivery catheter 90 is percutaneously and transluminally advanced along guide wire 110 until tip 83 of the catheter is disposed within lesion L within body vessel V, for example, as determined by fluoroscopic imaging. Once balloon 82 is positioned adjacent lesion L, sheath 92 is retracted proximally until radio-opaque marker 108 on sheath 92 is aligned with marker 105 of inner member 80, thereby indicating that the sheath has been retracted clear of balloon 82, as shown in FIG. 8B.

With respect to FIG. 8C, once balloon 82 is positioned adjacent lesion L, the balloon may be inflated to dilate a portion of the vessel and disrupt the plaque comprising lesion L. Balloon 82 then may be deflated, moved to another location within the lesion, and re-inflated to disrupt another portion of lesion L. This process is repeated until the lesion has been sufficiently disrupted to restore patency to the vessel.

Referring to FIG. 8D, after performing angioplasty, delivery catheter 90 is advanced so that balloon 82 is disposed adjacent healthy tissue, distal of the lesion. Balloon 82 then is inflated to engage the vessel wall and prevent axial displacement of the delivery catheter during subsequent retraction of sheath 92. Polymer layer 87 engages distal portion 32 of vascular prosthesis 28, thereby preventing axial displacement of distal portion 32 during retraction of sheath 92.

With respect to FIG. 8E, after balloon 82 is inflated to engage the vessel wall, sheath 92 is retracted proximally until distal portion 32 self-expands into engagement with vessel wall within or distal to lesion L. Proximal movement of sheath 92 may be halted once radio-opaque marker 108 of sheath 92 is substantially aligned with radiopaque marker 106 of inner member 80. When released from the constraint provided by sheath 92, the struts of distal portion 32 expand in a radial direction to engage the interior of vessel V. Stress relieving articulation, comprising connection members 112a and 112b, permit distal portion 32 to engage with the wall of vessel V while mitigating torsional forces applied to the distal edge of helical body portion 30.

Referring now to FIG. 8F, after distal portion 32 is secured to the vessel wall distal of lesion L, sheath 92 is further retracted proximally to cause the helical body portion of stent 28 to unwind and deploy to its predetermined shape within vessel V. During proximal retraction of sheath 92, each subsequent turn unwinds one at a time and engages and conforms to an inner wall of vessel V in a controlled manner.

Torsional forces applied to distal portion 32 during retraction of sheath 92 are uniformly distributed over the surface of balloon 82, thereby reducing the risk of insult to the vessel endothelium. Once the last turn of the helical body portion of stent 28 is deployed, balloon 82 is deflated, and the sheath optionally may be advanced to cover balloon 82. Delivery catheter 90 then is withdrawn from the patient's vessel, and guide wire 110 is removed, completing the procedure.

While the delivery catheter of FIG. 7 is depicted as employing balloon 82, it should be understood that the use of such a balloon is not limiting. Accordingly, vascular prostheses may be readily delivered using delivery systems comprising only the sheath and inner member components described with respect to FIG. 4. In that case, operation of the catheter to deliver the vascular prosthesis of the present invention would be as described with respect to FIGS. 8E through 8G.

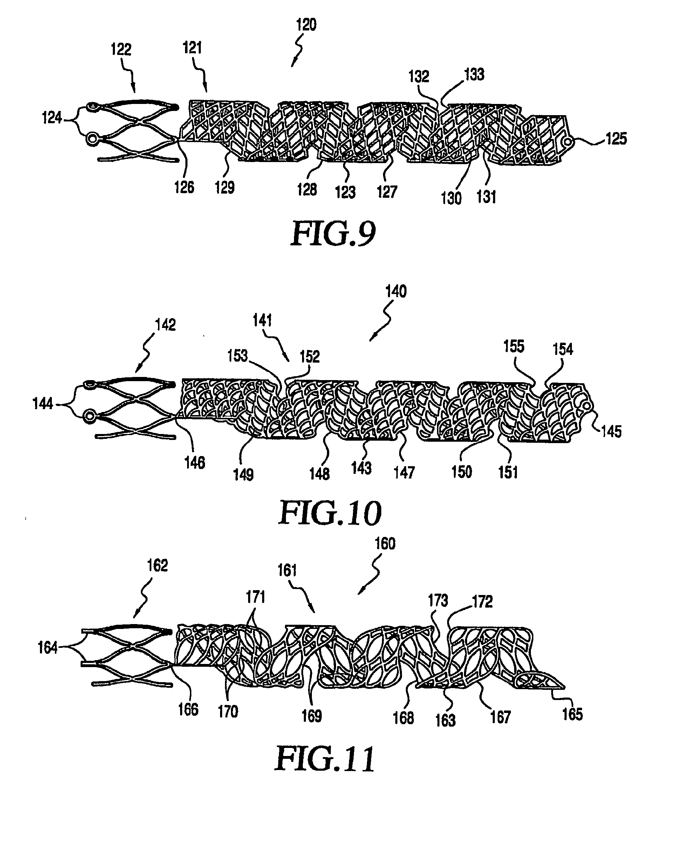

Referring now to FIGS. 9 to 11, further alternative examples of vascular prostheses are described. In each of FIGS. 9 through 11, the prostheses are depicted in the deployed configuration.

Vascular prosthesis 120 of FIG. 9 comprises helical body portion 121 and distal portion 122, which are connected by junction 126. Helical body portion 121 comprises a number of turns 123 having proximal edge 127 and distal edge 128. Body 121 further comprises proximal anchor point 125, whereas distal portion 122 comprises distal anchor point 124. Cells 129 have a polygonal shape, substantially resembling parallelograms. Struts 131 vary in shape around cell 129, such that hinges 130 are substantially aligned along the longitudinal axis of vascular prosthesis 120 when the device is in a deployed configuration. A series of crests 132 and troughs 133 extend along proximal edge 127 and distal edge 128. Nesting of the stent in the delivery configuration is substantially as described above, with crests 132 of each outer layer preferably disposed adjacent to troughs 133 of the adjacent underlying layer, and troughs 133 of each outer layer disposed adjacent to crests 132 of the adjacent underlying layer.

In FIG. 10, vascular prosthesis 140 comprises helical body portion 141 and distal portion 142, which are connected by junction 146. Helical body portion 141 comprises a number of turns 143 having proximal edge 147 and distal edge 148. Body 141 further comprises proximal anchor point 145, whereas distal portion 142 comprises distal anchor point 144. Cells 149 have a scallop shape, in that struts 151 are arched and joined together at hinges 150. As a result, distal edge 148 comprises an alternating series of crests 152 and valleys, whereas proximal edge comprises an alternating series of troughs 153 and points 155. Nesting of the stent 140 is accomplished by winding the stent such that troughs 153 and points 155 of one layer are adjacent to crests 152 and valleys 154, respectively, in an adjacent layer.

In FIG. 11, vascular prosthesis 160 comprises helical body portion 161 and distal portion 162, which are connected by junction 166. Helical body portion 161 comprises a number of turns 163 having proximal edge 167 and distal edge 168. Body 161 further comprises proximal anchor point 165, whereas distal portion 162 comprises distal anchor point 164. Struts 171 have a sinusoidal shape and alternate in phase. As a result, cells 169 have either a roughly diamond shape or convex shape. Distal edge 168 and proximal edge 167 each comprises an alternating series of crests 172 and troughs 173. Nesting of the stent in a delivery configuration preferably is accomplished by winding the stent so that crests 172 of each outer layer is disposed adjacent to troughs 173 of the adjacent underlying layer and troughs 173 of each outer layer is disposed adjacent to crests 172 of the adjacent underlying layer.

Although the preferred examples described herein above include a distal portion, it should be understood that the presence of the distal portion is not necessary to proper functioning with respect to obtaining improved flexibility or nesting. Accordingly, it should be understood that these features may be advantageously employed in helical stents that omit a distal portion as described above, and that the appended claims are intended to cover such prostheses.

While preferred illustrative embodiments of the invention are described above, it will be apparent to one skilled in the art that various changes and modifications may be made therein without departing from the invention as disclosed in the appended claims.

## Claims

1. A method of winding a vascular prosthesis (10; 28; 120; 140; 160) comprising:

   providing a vascular prosthesis comprising a helical body portion (14; 30; 30'; 121; 141; 161), the helical body portion comprising an elongated member disposed along a helical path about a longitudinal axis (40; 40') of the prosthesis to form a helix, the elongated member comprising a plurality of cells (36; 36'; 129; 149; 169), each cell having an open region and one or more struts (16; 131; 151; 171), the helical body portion having a reduced delivery configuration wherein one or more turns (23; 123; 143; 163) of the helical body portion overlap, **characterized by**
   winding the helical body portion to the reduced delivery configuration so that a strut of a first turn of the helix nests within an open region of a cell of a second turn of the helix with the first turn overlapping the second turn.

2. The method of claim 1, wherein winding the helical body portion (14; 30; 30'; 121; 141; 161) to the reduced delivery configuration comprises spacing the overlapping turns (23; 123; 143; 163) so that each turn is axially offset a predetermined distance from an adjacent overlapping turn.

## Patentansprüche

1. Verfahren zum Wickeln einer vaskulären Prothese (10; 28; 120; 140; 160) mit den folgenden Schritten:

   Bereitstellen einer vaskulären Prothese mit einem spiralförmigen Körperabschnitt (14; 30; 30'; 121; 141; 161), der ein längliches Element entlang einer spiralförmigen Strecke um eine Längsachse (40; 40') der Prothese zur Ausbildung einer Spirale aufweist, wobei das längliche Element eine Mehrzahl von Zellen (36; 36'; 129; 149; 169) aufweist, die jeweils einen offenen Bereich sowie eine oder mehr Streben (16; 131; 151; 171) aufweisen, wobei der spiralförmige Körperabschnitt eine verminderte Zuführkonfiguration aufweist, bei der sich ein oder mehr Windungen (23; 123; 143; 163) des spiralförmigen Körperabschnitts überschneiden, **gekenn-**

**zeichnet durch** den folgenden Schritt:

Wickeln des spiralförmigen Körperabschnitts in die verminderte Zuführkonfiguration, so dass sich eine Strebe einer ersten Windung der Spirale in einem offenen Bereich einer Zelle einer zweiten Windung der Spirale befindet, wobei die erste Windung die zweite Windung überschneidet.

2. Verfahren nach Anspruch 1, wobei der Schritt des Wickelns des spiralförmigen Körperabschnitts (14; 30; 30'; 121; 141; 161) in die verminderte Zuführkonfiguration ein Beabstanden der sich überschneidenden Windungen (23; 123; 143; 163) umfasst, so dass jede Windung zu einer benachbarten überschneidenden Windung um eine bestimmte Strecke axial versetzt ist.

**Revendications**

1. Procédé pour enrouler une prothèse vasculaire (10 ; 28 ; 120 ; 140 ; 160) comprenant l'étape consistant à :

prévoir une prothèse vasculaire comprenant une partie de corps hélicoïdal (14 ; 30 ; 30' ; 121 ; 141 ; 161), la partie de corps hélicoïdal comprenant un élément allongé disposé le long d'une trajectoire hélicoïdale autour d'un axe longitudinal (40 ; 40') de la prothèse afin de former une hélice, l'élément allongé comprenant une pluralité de cellules (36 ; 36' ; 129 ; 149 ; 169), chaque cellule ayant une région ouverte et une ou plusieurs entretoises (16 ; 131 ; 151 ; 171), la partie de corps hélicoïdal ayant une configuration de pose réduite dans laquelle une ou plusieurs spires (23 ; 123 ; 143 ; 163) de la partie de corps hélicoïdal se chevauchent, **caractérisé par** l'étape consistant à :

enrouler la partie de corps hélicoïdal dans la configuration de pose réduite de sorte qu'une entretoise d'une première spire de l'hélice s'emboîte à l'intérieur d'une région ouverte d'une cellule d'une deuxième spire de l'hélice avec la première spire qui chevauche la deuxième spire.

2. Procédé selon la revendication 1, dans lequel l'étape consistant à enrouler la partie de corps hélicoïdal (14 ; 30 ; 30' ; 121 ; 141 ; 161) dans la configuration de pose réduite comprend l'étape consistant à espacer les spires (23 ; 123 ; 143 ; 163) qui se chevauchent de sorte que chaque spire est axialement décalée selon une première distance d'une spire chevauchante adjacente.

## FIG.1

## FIG.2A

**FIG.2B**

**FIG.3**

FIG.4

FIG.5

FIG.6

**FIG.7**

**FIG.8A**

**FIG.8B**

**FIG.8C**

13

FIG.8D

FIG.8E

FIG.8F

FIG.8G

**FIG.9**

**FIG.10**

**FIG.11**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0062711 A, Rivelli
- US 5575816 A
- US 5906639 A, Rudnick
- US 6425915 B, Khosravi
- WO 2004058100 A